# EUROPEAN PATENT APPLICATION

(11) **EP 2 687 509 A2**
(43) Date of publication of application: **22.01.2014**
(21) Application number: 13176421.9
(22) Date of filing: 13.07.2013
(51) Int. Cl.: C07D 209/76, A61K 31/4035, A61P 35/00

(54) **Dicarboxyimides derivatives for use in the treatment of cancer**

(30) Priority: 16.07.2012 PL 40000012
(71) Applicant: Warszawski Uniwersytet Medyczny, 02-091 Warszawa (PL); Centrum Badan Molekularnych I Makromolekularnych Pan, 90-363 Lodz (PL)
(72) Inventor: Kuran, Bozena, 96-320 Mszczonów (PL); Krawiecka, Mariola, 28-200 Staszów (PL); Kossakowski, Jerzy, 04-854 Warszawa (PL); Cieslak, Marcin, 93-640 Lódz (PL); Kazmierczak-Baranska, Julia, 94-123 Lódz (PL); Królewska Karolina, 94-003 Lódz (PL); Nawrot, Barbara, 95-100 Dabrówka Wielka (PL)
(74) Representative: Brodowska, Iwona

(57) **Abstract**

The subject of the invention covers the substituted derivatives of dicarboximides represented by the general formula 1: 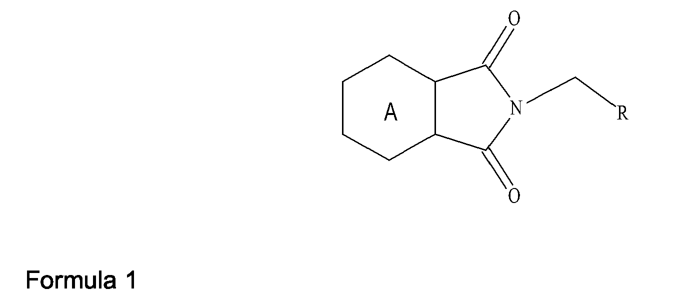 possibly in the form of pharmaceutically acceptable salts, wherein R means the alkyl group C1-C6, optionally, the alkyl group is substituted with -OH group or amine group, selected from group containing -NH-R₁ group or the group with formula 2 or 3, where R₁, R₂, R₃ are the same or different and cover the alkyl group C1-C3 and R₄ means -CH₂- or -O- 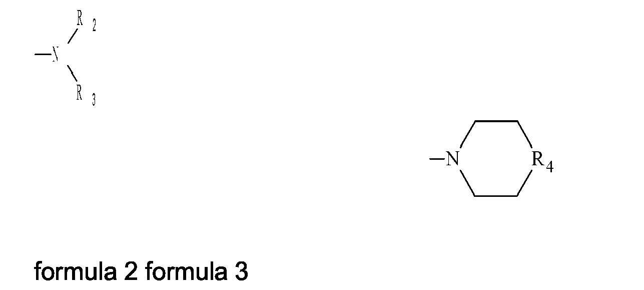 and the application of these compounds, or in combination with other drugs.

## Description

### Technical Field

The subject of the invention covers the substituted derivatives of dicarboximides represented by the general formula 1:

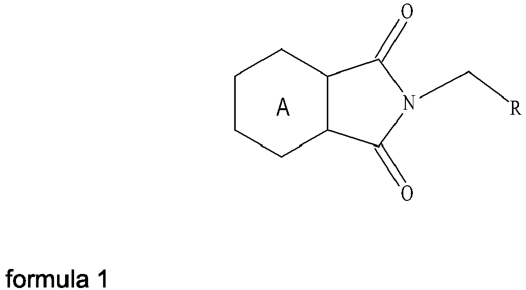

optionally in the form of pharmaceutically acceptable salts, wherein R means the alkyl group C1-C6, optionally, the alkyl group is substituted -OH group or amine group, selected from group containing -NH-R₁ group or the group with formula **2** or formula **3**, where R₁ ,R₂, R₃ are the same or different and cover the alkyl group C1-C3 and R₄ means -CH₂- or -O-

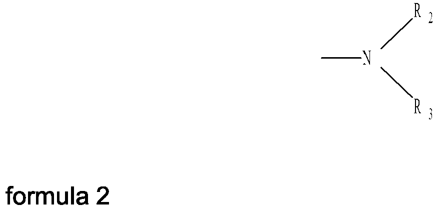

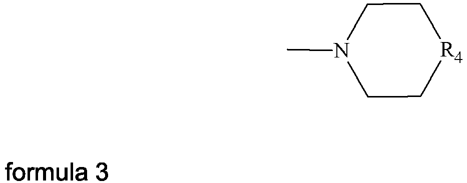

and the application of these compounds, or in combination with other drugs.

### Background Art

Imides derivatives are a broad group of compounds with multiple biological properties. Published data indicate that these compounds can be used as anti-neoplastic compounds, in the treatment of diabetes or as inhibitors of necrosis induced by oxidative stress. The first clinical trials of naphtalenoimides derivatives, amonafide and mitonafide as anti-neoplastic compounds were carried out in the 80's of the last century (1-2). Although in the phase II and phase III clinical studies these compounds showed anti-cancer activity in patients with leukemia and solid tumor cancers, further clinical trials have been suspended because of serious side effects, including neurotoxicity. Cytotoxicity of these compounds resulted mainly from the intercalation into DNA and inhibition of topoisomerase II activity. Using mitonafide as a lead compound, a series of mono- and bis-2-(2-(dimethylamino)-ethyl)-5-nitro-1 H-benzo[de]izoquinolino-1,3(2H)-d ione with the various amine substituents at position 6 was designed and synthesized. With the aid of MTT and SRB assays cytotoxicity of these derivatives was determined in human tumor cell lines HeLa, A549, P388, HL-60, MCF-7, HCT-8 and A375. Cytotoxicity of these derivatives was similar to mitonafide, with IC₅₀ values falling within the range of 10⁻⁶ - 10-5 M (3). In turn, a derivative of 6-nitro-2-(3-hydroxypropyl)-1H-benz[de]izoquinolino-1,3-dione was toxic to 8 of 13 tested tumor cell lines (leukemia, breast cancer, neuroblastoma, colorectal cancer, liver, prostate, lung). Moreover, this compound showed a low toxicity for normal cells (peripheral blood lymphocytes, IC₅₀ 273µM) (4). Using MOLT-4 (lymphoblastic leukemia) and HL-60 (promyelocytic leukemia) cells it was demonstrated that this compound induces apoptosis and cell cycle arrest in the sub-G1 and G2/M phases. Incubation of MOLT-4 cells with the test compound at a concentration of 5µM caused a significant increase of caspase-3 activity (after 12 hours of incubation), and caspase-6 (after 24 hours of incubation). An interesting biological activity has been observed for the derivative of amonafide: 2,2,2-trichloro-N-({2-[2-(dimethylamino)ethyl]-1,3-dioxo-2,3-dihydro-1H-be nzo[de]izoquinolino-5-ylo}carbamoylo)acetamide (UNBS3157). This compound is highly toxic to cancer cells but the mechanism of toxicity is quite different than expected. UNBS3157 induces autophagy and aging (senscence) of tumor cells (5). The maximum tolerated dose for this compound is about 4x higher than for the lead compound - amonafide. Moreover, when administered to mice at doses inducing antitumor effect, this compound showed no haemotoxicity. The test results obtained withinin *in vivo* models of murine leukemia, breast cancer and orthotopic models of non-small cell lung cancer and pancreatic cancer indicate enhanced activity of UNBS3157 compared to amonafide. In studies carried out by Chen et al. (6) a new class of naphtalenoimide derivatives was designed and synthesized. These compounds were characterized by a higher tumor cell cytotoxicity (IC₅₀ in the range of 2-10 µM) than amonafide. Interestingly, these compounds proved to be weak intercalators of DNA, and their cytotoxicity resulted from inhibition of topoisomerase II activity, lysosomal membrane permeabilization and induction of apoptosis through the mitochondrial pathway.

Another interesting group of compounds are indolomaleimide derivatives. These compounds evoked a great interest due to the antitumor properties arising from the ability to intercalate into DNA, induction of apoptosis, cell cycle arest, inhibition of protein kinase C (PKC). Xu et al. (7) synthesized and examined antitumor activity of 4-chloro-3-arylmaleimide derivatives on leukemia cells (HL60), prostate cancer (PC-3), non-small cell lung cancer (A549), liver cancer (SMMC-7721), and gastric cancer (SGC-7901). The highest cytotoxicity was displayed by a compound having a bromine atom in the 5-position of the indole ring. For HL60 cells, IC₅₀ parameter for this compound was 1.5 nM.

PKC is a family of serine-threonine kinases consisting of 15 isozymes which can be arranged into a few classes, such as: α, β, γ, δ, ε, λ, η. PKC kinases regulate a number of processes, such as cell proliferation and gene expression. The compounds that selectively inhibit the activity of specific PKC isozymes may have a therapeutic value in the treatment of cancer or diabetes. Research by Faul et al. (8) indicates that the acyclic N-(azacycloalkyl)bisindolomaleimides are PKCβ inhibitors (IC₅₀ in the range of 10⁻⁹ - 10⁻⁶ M). The selectivity of these inhibitors towards PKC is about 300 times greater than for calmodulin-dependent kinase and about 3000 times greater than for src kinase. In turn research by Sanchez-Martinez et al. (9) demonstrates that the bis-indolomaleimides and indolocarbazoles are inhibitors of cyclin-dependent kinases (CDKs). These kinases are key regulators of the cell cycle, and compounds that inhibit the activity of these enzymes may be used in the treatment of cancer. In studies on inhibitors of CDK4 and CDK2 it has been observed that indolocarbazoles are better inhibitors compared to the bis-indolomaleimides. The compounds that are inhibitors of CDK2 and CDK4 are also cytotoxic to tumor cells (line HCT-116 and NCI H460), and cause cell cycle arrest in the G1 and the G2/M phases. Sodeoka et al. (10) described interesting properties of bis-indolomaleimide derivatives. They identified compounds that are selective inhibitors of necrosis induced by oxidative stress. Further studies have shown that these compounds do not block the process of caspase-dependent apoptosis. It is considered that the derivatives can be used as cardioprotective compounds during reperfusion of hypoxic myocardial cells.

### Summary of invention

New substituted derivatives of dicarboximides, represented by the general formula 1:

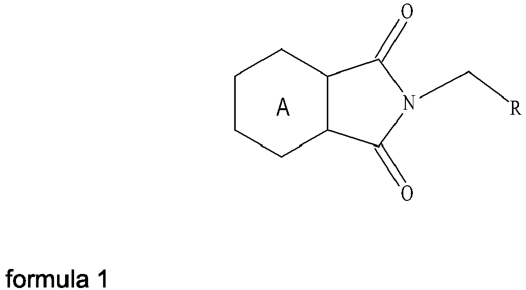

possibly in the form of pharmaceutically acceptable salts, wherein R means the alkyl group C1-C6, optionally, the alkyl group is substituted with the hydroxyl or amine group, selected from group containing -NH-R₁ substituent or the group with formula **2** or **3**, where R₁,R₂,R₃ are the same or different and cover the alkyl group C1-C3 and R₄ means -CH₂- or-O-

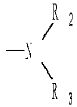

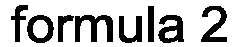

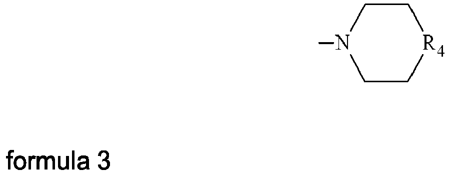

and combinations of these substituents, where **A** in Formula **1** means the structure represented by the general formula **a**

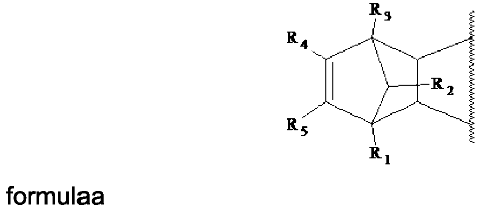

wherein R₁, R₃, R₄, R₅ are the same or different and mean the alkyl group-(CH₂)ₙCH₃ where n = 0 ÷ 6, or -C₆H₅ group and its derivatives selected from the group consisting halogen derivatives, hydroxyl derivatives, -O-alkyl type -O-(CH₂)ₙ-CH₃ where n = 0 ÷ 6,

R₂ means a -H, =O, -OH, -(CH₂)ₙCH₃ type substituents where n = 0 ÷ 6 or -C₆H₅ group and its derivatives selected from the group consisting halogen derivatives, hydroxyl derivatives, -O-alkyl type -O-(CH₂)ₙ-CH₃ where n = 0 ÷ 6,

or represented by the general formula **b**

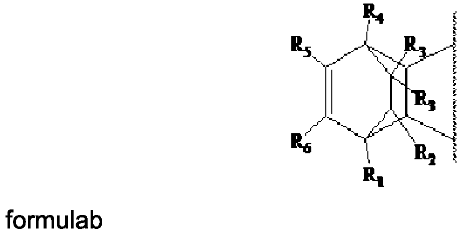

wherein:

R₁ means a -(CH₂)ₙCH₃, -O-(CH₂)ₙCH₃, -CO(CH₂)ₙCH₃ type substituents where

n = 0 ÷ 6,

R₂, R₄ are the same or different and mean a -H, -(CH₂)ₙCH₃, -O-(CH₂)ₙ CH₃ type substituents where n = 0 ÷ 6,

R₃, R₃ₐ, R₅, R₆ are the same or different and mean a -H, -OH,-halogen, -(CH₂)ₙCH₃ type substituents where n = 0 ÷ 6,

or represented by the general formula c

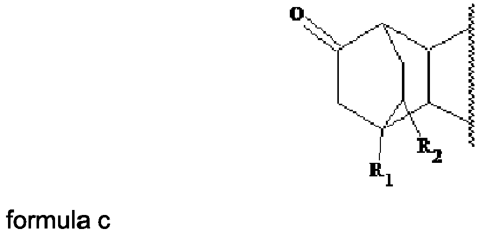

wherein:

R₁ means a -(CH₂)ₙCH₃, -O-(CH₂)ₙCH₃, -CO(CH₂)ₙCH₃ type substituents where

n = 0 ÷ 6,

R₂ means a -halogen, -(CH₂)ₙCH₃, -COO(CH₂)ₙCH₃ type substituents where n = 0 ÷ 6,

and their salts showing biological activity.

The examples of preferable compounds described by the general formula 1 and general structure **a**, as used herein, are described by formulas **a3**, **a4, a5, a6, a7 (examples I-V).**

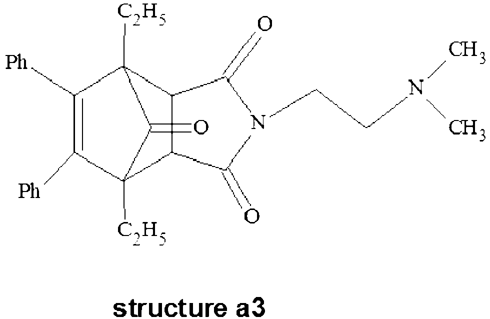

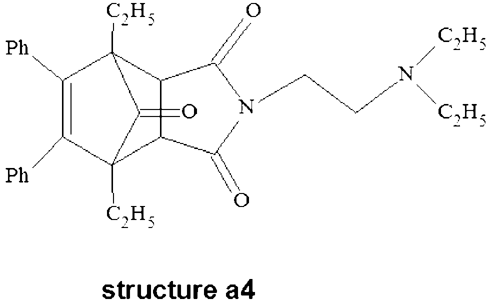

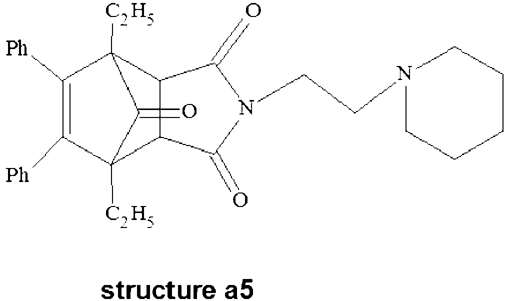

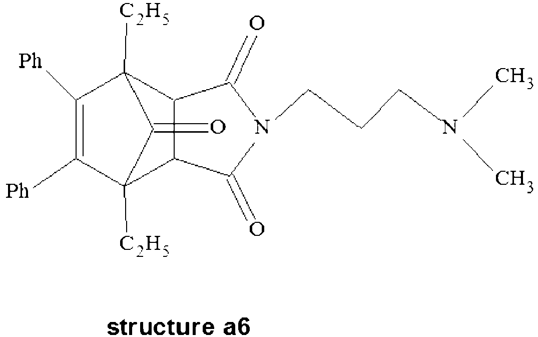

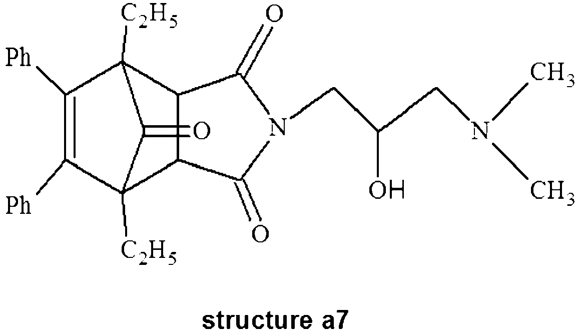

The compounds **a3-a7** (**a**: R₁= R₃=C₂H₅, R₂= =O, R₄= R₅= Phe) are highly toxic to the cancer cells of chronic myelogenous leukaemia K562 and acute myelogenous HL60. The IC₅₀ parameter (concentration of compound leading to 50% cell mortality) was determined by MTT test. After 48 hours of incubation of K562 and HL60 cells with compounds **a3-7**, the IC₅₀ values ranged from *1* to **10µM**. Under the same experimental conditions, compounds **a3-7** displayed virtually zero toxicity against normal HUVEC cells (IC₅₀ > 1 mM) and uterine cervix cancer cells HeLa (IC₅₀ > 1 mM). For comparison, IC₅₀ value for cis-platinum (compound used in neoplastic disease treatment) against K562 cells ranged from several to several dozens µM. Furthermore, subsequent experiments demonstrated that the incubation of compound **a5** with K562 cells (a5 concentration = 10 µM; incubation time = 24h) increased the activity of caspases 3 and 7, i.e. proteolytic enzymes being apoptosis markers. This indicates that the cytotoxicity of compound **a5** is related to induction of apoptosis inside the cancer cells. Selective toxicity of compound **a5** against the chronic and acute myelogenous leukaemia cells, absence of toxicity against normal cells, and apoptosis induction inside the cancer cells indicate that compound **a5** would be useful for the production of anti-neoplastic drugs or drugs against diseases of proliferative nature.

In order to obtain alkylamino dicarboximides of the formula 1, the appriopriate dicarboximide was condensed with halogenoalkylamine. For the synthesis of aminoalkanoles derivatives of dicarboximides, in the first step halogenoepoxide was used and then obtained product was condensed with an appropriate amine.

All obtained dicarboximide derivatives were converted to their hydrochlorides and crystallized from methanol/diethyl ether.

### Description of embodiments

**Examples of the implementation of the invention.**

**General conditions of the synthesis of N-alkylamino derivatives of dicarboximides**

**(Example I-X)**

The appriopriate imide: 1,7,8,9-tetraphenyl-4-azatricyclo[5.2.1.0^{2,6} ]dec-8-ene-3,5-dione / 1,7-diethyl-8,9-diphenyl-4-azatricyclo[5.2.1.0^{2,6} ]dec-8-ene-3,5,10-trione was dissolved in acetone (30 mL), then anhydrous K₂CO₃ (0.01 mol), a catalytic amount of 98% 1,8-diazabicyclo[5.4. 0]undec-7-ene (DBU) and an appropriate halogenoalkilamine (0.01 mol) were added. The reaction was carried out in reflux, respectively for 8-14 hours. Reaction was monitored by TLC. The solvent was evaporated and the residue was purified by column chromatography on silica gel (eluent: chloroform or chloroform/methanol 50:0.2).

**Example I (a 1)**

**R1=-Phe, R₂ = H, R₃ =-Phe, R₄ =-Phe, R₅ =-Phe, R =-CH₂N(CH₃)₂**

Yield: 82%; white powder, m.p. 269-271 °C (from EtOH/(Et)₂O); ¹H NMR (300MHz, DMSO-d₆+ TMS, δ/ppm): 2.40 (1H, *d*, *J*=8.4, C10-H), 2.83 (6H, *m,* -CH₃), 3.29 (3H, *m*, C1'-H, C10-H), 3.73 (2H, *t*, *J*=5.5, C2'-H), 4.32 (2H, s, C2-H, C6-H), 6.48 (4H, *m*, Ar-H), 6.92 (6H, *m*, Ar-H), 7.30 (6H, *m,* Ar-H), 7.75 (4H, *d*, *J*=7.2, Ar-H), 10.01 (1 H, *s*, HCl); MS (m/z): 100% = 539.2, 65% = 540.3 [L+H⁺].

**Example II**

**R₁= -Phe, R₂ = H, R₃ = -Phe, R₄ = -Phe, R₅ = -Phe,R= -CH₂N(C₂H₅)₂**

Yield: 89 %; white powder, m.p. 263-264.1 °C (from EtOH/(Et)₂O); ¹H NMR (300MHz, DMSO-d₆+ TMS, δ/ppm): 1.20 (6H, *t*, *J*=7.2, -CH₃), 2.37 (1 H, *m,* C10-H), 3.20 (7H, *m*, C1'-H, C10-H, -CH₂-), 3.72 (2H, *m*, C2'-H), 4.31 (2H, *s*, C2-H, C6-H), 6.51 (4H, *d*, *J*=6.9, Ar-H), 6.92 (6H, *m*, Ar-H), 7.30 (6H, *m*, Ar-H), 7.75 (4H, *d, J*=7.2, Ar-H), 9.75 (1 H, s, HCl); MS (m/z): 100% = 567.3, 55% = 568.4 [L+H⁺].

**Example III**

**R₁= -Phe, R₂ = H, R₃ = -Phe, R₄ = -Phe, R₅ = -Phe,R= -CH₂NC₄H₈O**

Yield: 76 %; white powder, m.p. 185.5-187 °C (from EtOH/(Et)₂O); ¹H NMR (300MHz, DMSO-d₆+ TMS, δ/ppm): 2.37 (1H, *d*, *J*=8.4, C10-H), 3.12 (2H, *m*, C1'-H), 3.45 (4H, *m*, C10-H, H-morph.), 3.76 (3H, *m*, C2'-H, H-morph.), 4.00 (4H, *m*, H-morph.) 4.33 (2H, *s*, C2-H, C6-H), 6.48 (4H, *m,* Ar-H), 6.91 (6H, *m*, Ar-H), 7.30 (6H, *m*, Ar-H), 7.75 (4H, *d, J*=7.2, Ar-H), 10.83 (1 H, *s,* HCl); MS (m/z): 100% = 581.3, 19% = 582.4 [L+H⁺].

**Example IV**

**R₁= -Phe, R₂ = H, R₃ = -Phe, R₄ = -Phe, R₅ = -Phe,R= -CH₂NC₅H₁₀**

Yield: 78 %; white powder, m.p. 257-258.5 °C (from EtOH/(Et)₂O); ¹H NMR (300MHz, DMSO-d₆+ TMS, δ/ppm): 1.40 (1 H, *m*, H-piper.), 1.72 (5H, *m*, H-piper.), 2.39 (1H, *m*, C10-H), 2.93 (2H, *m*, H-piper.), 3.24 (3H, *m*, C10-H, C1'-H), 3.57 (2H, *m*, H-piper.), 3.74 (2H, *t*, *J*=6.4, C2'-H), 4.32 (2H, s, C2-H, C6-H), 6.51 (4H, *m*, Ar-H), 6.92 (6H, *m*, Ar-H), 7.30 (6H, *m*, Ar-H), 7.75 (4H, *d*, *J*=7.2, Ar-H), 9.69 (1 H, s, HCl); MS (m/z): 100% = 579.3, 72% = 580.3 [L+H⁺].

**Example V (a 2)**

**R₁= -Phe, R₂ = H, R₃ = -Phe, R₄ = -Phe, R₅ = -Phe,R= -(CH₂)₂N(CH₃)₂**

Yield: 80 %; white powder, m.p. 246-247.5 °C (from EtOH/(Et)₂O); ¹H NMR (300MHz, DMSO-d₆+ TMS, δ/ppm): 1.86 (2H, m, C2'-H), 2.35 (1H, *d* , *J*=8.4, C10-H), 2.70 (6H, *m*, -CH₃), 3.07 (2H, *m*, C1'-H), 3.17 (1 H, *s*, C10-H), 3.43 (2H, *t*, *J*=6.7, C3'-H), 4.28 (2H, *s*, C2-H, C6-H), 6.49 (4H, *m*, Ar-H), 6.92 (6H, *m*, Ar-H), 7.27 (6H, *m*, Ar-H), 7.76 (4H, *d*, *J*=7.2, Ar-H), 9.82 (1H, *s*, HCl); MS (m/z): 100% = 553.3, 70% = 554.3 [L+H⁺].

**Example VI (a3)**

**R₁= -C₂H₅, R₂ = =O, R₃ = -C₂H₅, R₄ = -Phe, R₅ = -Phe, R= -CH₂N(CH₃)₂**

Yield: 82 %; white powder, m.p. 238-239 °C (from EtOH/(Et)₂O); ¹H NMR (300MHz, DMSO-d₆+ TMS, δ/ppm): 0.88 (6H, *t*, *J*=7.3, -CH₃), 1.87 (2H, *m*,
- CH₂-), 2.07 (2H, *m*, -CH₂-), 2.78 (6H, *s*, N-CH₃), 3.27 (2H, *m*, C1'-H), 3.72 (2H, *s*, C2-H, C6-H), 3.82 (2H, *t*, *J*=5.7, C2'-H), 6.86 (4H, *m*, Ar-H), 7.18 (6H, *m*, Ar-H), 10.50 (1 H, *s*, HCl); MS (m/z): 100% = 457.3, 34% = 458.3 [L+H⁺].

**Example VII (a4)**

**R₁= -C₂H₅, R₂ = =O, R₃ = -C₂H₅, R₄ = -Phe, R₅ = -Phe, R= -CH₂N(C₂H₅) ₂**

Yield: 88 %; white powder, m.p. 217-218 °C (from EtOH/(Et)₂O); ¹H NMR (300MHz, DMSO-d₆+ TMS, δ/ppm): 0.89 (6H, *t*, *J*=7.3, -CH₃), 1.19 (6H, *t*, *J*=7.0, -CH₃), 1.88 (2H, *m*, -CH₂-), 2.08 (2H, *m*, -CH₂-), 3.19 (6H, *m*, C1'-H, -CH₂-CH₃), 3. 17 (2H, *s*, C2-H, C6-H), 3.80 (2H, *t*, *J*=6.7, C2'-H), 6.87 (3H, *m*, Ar-H), 7.19 (5H, *m*, Ar-H), 9.89 (1 H, *s*, HCl); MS (m/z): 100% = 485.3, 26% = 486.3 [L+H⁺].

**Example VIII**

**R₁= -C₂H₅, R₂ = =O, R₃ = -C₂H₅, R₄ = -Phe, R₅ = -Phe, R= -CH₂NC₄H₈O**

Yield: 84 %; white powder, m.p. 230-232 °C (from EtOH/(Et)₂O); ¹H NMR (300MHz, DMSO-d₆+ TMS, δ/ppm): 0.88 (6H, *t*, *J*=7.3, -CH₃), 1.87 (2H, *m*, -CH₂-), 2.07 (2H, *m*,-CH₂-), 3.12 (2H, *m*, C1'-H), 3.49 (4H, *m*, H-morph.), 3.72 (2H, *s*, C2-H, C6-H), 3.79 (4H, *m*, H-morph.), 3.98 (2H, *m*, C2'-H), 6.86 (4H, *m*, Ar-H), 7.18 (6H, *m*, Ar-H), 11.10 (1H, *s*, HCl); MS (m/z): 100% = 499.3, 27% = 500.3 [L+H⁺].

**Example IX (a5)**

**R₁= -C₂H₅, R₂ = =O, R₃ = -C₂H₅, R₄ = -Phe, R₅ = -Phe, R= -CH₂NC₅H₁₀**

Yield: 79 %; white powder, m.p. 235-238 °C (from EtOH/(Et)₂O); ¹H NMR (300MHz, DMSO-d₆+ TMS, δ/ppm): 0.88 (6H, *t*, *J*=7.5, -CH₃), 1.38 (1H, *m*, H-piper.), 1.71 (5H, *m*, H-piper.), 1.87 (2H, *m*, -CH₂-), 2.08 (2H, *m*, -CH₂-), 3.19 (2H, *m*, H-piper.), 3.51 (2H, *m*, C1'-H), 3.72 (2H, *s*, C2-H, C6-H), 3.84 (2H, *t*, *J*=6.3, C2'-H), 6.86 (4H, *m*, Ar-H), 7.18 (6H, *m*, Ar-H), 10.08 (1H, *s*, HCl); MS (m/z): 100% = 497.3, 47% = 498.4 [L+H⁺].

**Example X (a6)**

**R₁= -C₂H₅, R₂ = =O, R₃ = -C₂H₅, R₄ = -Phe, R₅ = -Phe, R= -(CH₂)₂N(CH₃ )₂**

Yield: 86 %; white powder, m.p. 215-217 °C (from EtOH/(Et)₂O); ¹H NMR (300MHz, DMSO-d₆+ TMS, δ/ppm): 0.89 (6H, *t*, *J*=7.5, -CH₃), 1.87 (4H, *m*, -CH₂-, C3'-H), 2.06 (2H, *m*, -CH₂-), 2.68 (6H, *s*, N-CH₃), 3.06 (2H, *m*, C1'-H), 3.49 (2H, *t, J*=7.0, C2'-H), 3.68 (2H, *s*, C2-H, C6-H), 6.87 (4H, *m*, Ar-H), 7.20 (6H, *m*, Ar-H), 10.23 (1 H, *s*, HCl); MS (m/z): 100% = 471.3, 54% = 472.3 [L+H⁺].

**General conditions of synthesis of alkylamine derivatives of dicarboximides**

**(Examples XI-XVIII)**

Step I:

The appriopriate imide: 1,7,8,9-tetraphenyl-4-azatricyclo[5.2.1.0^{2,6} ]dec-8-ene-3,5-dione / 1,7-diethyl-8,9-diphenyl-4-azatricyclo[5.2.1.0^{2,6} ]dec-8-ene-3,5,10-trione / 1,7,8,9-tetraphenyl-4-azatricyclo[5.2.1.0^{2,6} ]dec-8-ene-3,5,10-trione / 1,7-dimethyl-8,9-diphenyl-4-azatricyclo[5.2.1.0 ^{2,6}]dec-8-ene-3,5,10-trione (0.01 mol) was dissolved in 1-chloro-2,3-epoxypropane (50-60 mL), then an anhydrous K₂CO₃ (0.01mol) was added. Reactions were carried out at room temperature on a magnetic stirrer under reflux with a tube with CaCl₂, for 15 h. When the reaction was completed (TLC control) the inorganic precipitate was filtered off and the filtrate was concentrated. The oily product was purified by column chromatography on- silica gel (eluent: chloroform and chloroform/methanol 50:0.2).

Step II:

The appropriate 4-(oxirane-2-ylmethyl)-4-imide (0.001 mol) was dissolved in 30 mL of methanol/water (29:1) solvent system. Reactions were carried out at room temperature on a magnetic stirrer under reflux for 15 - 20 hours. When the reaction was completed (TLC control), the excess of the solvent was evaporated and the crude product was purified by column chromatography on silica gel (eluent: chloroform or chloroform/methanol (50:0.2, 50:0.5)).

**Example XI (a9)**

**R₁= -Phe, R₂ = H, R₃ = R₄ = R₅ = -Phe, R = -CHOHCH₂NHCH(CH₃)₂**

Yield: 92 %; white powder, m.p. 169-176 °C (from EtOH/(Et)₂O); ¹H NMR (300MHz, DMSO-d₆+ TMS, δ/ppm): 1.21 (6H, *m*, -CH₃), 2.36 (1H, *m*, C8-H), 2.85 (1H, *m*, -CH-), 3.06 (1 H, *m*, C2'-H), 3.26 (3H, *m*, C8-H, C1'-H), 3.50 (1H, *m*, C3'-H), 4.12 (1 H, *m*, C3'-H), 4.29 (2H, *s*, C2-H, C6-H), 5.77 (1H, *d*, *J*=4.8, OH), 6.49 (4H, *m*, Ar-H), 6.89 (6H, *m*, Ar-H), 7.27 (6H, *m*, Ar-H), 7.75 (4H, *d*, *J*=7.2, Ar-H), 8.38 (1 H, s, HCl); MS (m/z): 100% = 583.1, 48% = 584.1 [L+H⁺].

**Example XII (a10)**

**R₁= -Phe, R₂ = H, R₃ = R₄ = R₅ = -Phe, R = -CHOHCH₂N(CH₃)₂**

Yield: 92 %; white powder, m.p. 243-248 °C (from EtOH/(Et)₂O); ¹H NMR (300MHz, DMSO-d₆+ TMS, δ/ppm): 2.36 (1H, *d*, *J*=8.7, C8-H), 2.73 (3H, *m*, -CH₃), 3.05 (1 H, *m*, C2'-H), 3.16 (1H, *m*, C8-H), 3.29 (2H, *m*, C1'-H), 3.46 (1 H, *m*, C3'-H), 4.16 (1 H, *m*, C3'-H), 4.30 (2H, *s*, C2-H, C6-H), 5.96 (1H, *d*, *J*=5.1, OH), 6.50 (4H, *m*, Ar-H), 6.91 (6H, *m*, Ar-H), 7.27 (6H, *m*, Ar-H), 7.75 (4H, *d*, *J*=7.2, Ar-H), 9.59 (1H, *s*, HCl); MS (m/z): 100% = 569.1, 45% = 570.1 [L+H⁺].

**Example XIII (a7)**

**R₁= -C₂H₅, R₂ = =O, R₃ = -C₂H₅, R₄ = R₅ = -Phe,R = -CHOHCH₂ NHCH(CH₃)₂**

Yield: 87 %; white powder, m.p. 206-207.8 °C (from EtOH/(Et)₂O); ¹H NMR (300MHz, DMSO-d₆+ TMS, δ/ppm): 0.89 (6H, *m*, -CH₃), 1.21 (6H, *m* , -CH₃), 1.86 (2H, *m*, -CH₂-), 2.09 (2H, *m*, -CH₂-), 2.84 (1H, *m*, C1'-H), 3.07 (1 H, *m*, C1'-H), 3.25 (2H, *m*, C2'-H, -CH-), 3.39 (1 H, *m*, C3'-H), 3.57 (1 H, *m*, C3'-H), 3.69 (2H, *s*, C2-H, C6-H), 5.76 (1H, *d, J*=4.8, OH), 6.88 (4H, *m*, Ar-H), 7.17 (6H, *m*, Ar-H), 8.38 (1H, *m*, NH), 8.54 (1H, *s*, HCl); MS (m/z): 100% = 501.2, 8% = 502.3 [L+H⁺].

**Example XIV (a8)**

**R₁= -C₂H₅, R₂ = =O, R₃ = -C₂H₅, R₄ = R₅ = -Phe, R= -CHOHCH₂N(CH₃)₂**

Yield: 72 %; white powder, m.p. 213-215.5 °C (from EtOH/(Et)₂O); ¹H NMR (300MHz, DMSO-d₆+ TMS, δ/ppm): 0. 09 (6H, *m*, -CH₃), 1.86 (2H, *m*, -CH₂-), 2.09 (2H, *m*, -CH₂-), 2.75 (6H, *m*, -CH₃), 3.05 (1 H, *m*, C1'-H), 3.20 (1 H, *m*, C1'-H), 3.31 (1 H, *m*, C2'-H), 3.38 (1 H, *m*, C3'-H), 3.52 (1 H, *m* , C3'-H), 3.70 (2H, *s*, C2-H, C6-H), 5.95 (1H, *d, J*=5.4, OH), 6.87 (4H, *m*, Ar-H), 7.17 (6H, *m*, Ar-H), 9.61 (1H, *s*, HCl); MS (m/z): 100% = 487.3, 39% = 488.3 [L+H⁺].

**Example XV (a11)**

**R₁= -CH₃, R₂ = =O, R₃ = -CH₃, R₄ = R₅ = -Phe,R = -CHOHCH₂NHCH(CH 3)₂**

Yield: 79 %; white powder, m.p. 135.1-138 °C (from EtOH/(Et)₂O); ¹H NMR (300MHz, DMSO-d₆+ TMS, δ/ppm): 1.21 (6H, *m*, -CH₃), 1.42 (6H, *d*, *J*=3.6, -CH₃), 2.85 (1 H, *m*, C1'-H), 3.03 (1 H, *m*, C1'-H), 3.25 (1 H, *m*, C2'-H), 3.39 (2H, *m*, C3'-H, -CH-), 3.53 (3H, *m*, C2-H, C6-H, C3'-H), 5.80 (1H, *d*, *J*=4.8, OH), 6.87 (4H, *m*, Ar-H), 7.19 (6H, *m*, Ar-H), 8.40 (1 H, *s*, NH), 8.74 (1 H, *s*, HCl); MS (m/z): 100% = 473.1, 29% = 474.2 [L+H⁺].

**Example** XVI **(a12)**

**R₁= -CH₃, R₂ = =O, R₃ = -CH₃, R₄ = R₅ = -Phe, R=-CHOHCH₂N(CH₃)₂**

Yield: 84 %; white powder, m.p. 155.5-156.5 °C (from EtOH/(Et)₂O); ¹H NMR (300MHz, DMSO-d₆+ TMS, δ/ppm): 1.42 (6H, *d,* J=3.3, -CH₃), 2.08 (3H, *d*, *J*=4.5, -CH₃), 2.77 (3H, *d*, *J*=4.2, -CH₃), 3.06 (1 H, *m*, C1'-H), 3.16 (1 H, *m*, C1'-H), 3.37 (2H, *m*, C2'-H, C3'-H), 3.49 (3H, *m*, C2-H, C6-H, C3'-H), 4.10 (1 H, *m*, OH), 6.87 (4H, *m*, Ar-H), 7.19 (6H, *m*, Ar-H), 9.62 (1 H, *s*, HCl); MS (m/z): 100% = 459.1, 25% = 460.1 [L+H⁺].

**Example XVII (a13)**

**R₁= -Phe, R₂ = =O, R₃ = R₄ = R₅ = -Phe,R = -CHOHCH₂NHCH(CH₃)₂**

Yield: 87 %; white powder, m.p. 195-196 °C (from EtOH/(Et)₂O); ¹H NMR (300MHz, DMSO-d₆+ TMS, δ/ppm): 1.21 (6H, *m*, -CH₃), 2.90 (1H, *m*, -CH-), 3.10 (1H, *m*, C2'-H), 3.23 (1 H, *m*, C1'-H), 3.43 (1 H, *m*, C1'-H), 3.62 (1H, *m*, C3'-H), 4.18 (1 H, *m*, C3'-H), 4.51 (2H, *s*, C2-H, C6-H), 5.84 (1H, *d*, *J*=4.8, OH), 6.68 (4H, *m*, Ar-H), 6.92 (6H, *m*, Ar-H), 7.34 (6H, *m*, Ar-H), 7.68 (4H, *m*, Ar-H), 8.48 (1 H, *s*, HCl); MS (m/z): 100% = 597.3, 18% = 598.3 [L+H⁺].

**Example XVIII (a14)**

**R₁= -Phe, R₂ = =O, R₃ = R₄ = R₅ = -Phe,R = -CHOHCH₂N(CH₃)₂**

Yield: 86 %; white powder, m.p. 185-187 °C (from EtOH/(Et)₂O); ¹H NMR (300MHz, DMSO-d₆+ TMS, δ/ppm): 2.74 (6H, *m*, -CH₃), 3.13 (2H, *m*, C1'-H, C2'-H), 3.41 (1 H, *m*, C1'-H), 3.57 (1 H, *m,* C3'-H), 4.20 (1 H, *m*, C3'-H), 4.52 (2H, *s*, C2-H, C6-H), 6.02 (1H, *d*, *J*=4.8, OH), 6.71 (3H, *m*, Ar-H), 6.96 (6H, *m*, Ar-H), 7.10 (1 H, *m*, Ar-H), 7.34 (6H, *m*, Ar-H), 7.67 (4H, *m*, Ar-H), 9.49 (1 H, *s*, HCl); MS (m/z): 100% = 583.3, 15% = 584.3 [L+H⁺].

**General conditions of synthesis of N-alkylamino derivatives of dicarboximides**

**Examples XIX-XLVIII**

An appriopriate imide, 1-methoxy-4-azatricyclo[5.2.2.0^{2,6} ]undec-8-ene-3,5-dione / 1,7-diacetyl-4-azatricyclo[5.2.2.0^{2,6} ]undec-8-ene-3,5-dione / 1,8,11,11-tetramethyl-4-azatricyclo[5.2.2.0^{2,6} ]undec-8-ene-3,5-dione / 8 ethyl ester 11-ethyl-10-hydroxy-7-methyl-3 ,5-dioxo-4-azatricyclo[5.2.2.0^{2,6}]undec-10-ene / 1-isobutoxy-4-azatricyclo[5.2.2.0^{2,6}]undec-3,5,8-trione / 8-ethyl ester 7-methyl-3,5,10-trioxo-4-azatricyclo[5.2.2.0^{2,6}]undecane was dissolved in acetone (30 mL), then anhydrous K₂CO₃ (0.01 mol), a catalytic amount of 98% 1,8-diazabicyclo[5.4.0]undec-7-ene (DBU) and the appropriate halogenalkylamine (0.01 mol) were added. Reaction was carried out in reflux for 8-14 hours. When the reaction was completed (TLC control), the solvent was evaporated. The crude product was purified by column chromatography on silica gel (eluent: chloroform and chloroform/methanol 50:0.2).

**Example XIX (b1)**

**R₁= -OCH₃, R₂ = R₃ = R₃ₐ = R₄ = R₅ = R₆= -H, R= -CH₂N(CH₃)₂**

Yield: 81 %; white powder, m.p. 196.7-198 °C (from EtOH/(Et)₂O); ¹H NMR (300MHz, DMSO-d₆+ TMS, δ/ppm): 1.21 (1H, *m*, C11-H), 1.38 (1H, *m*, C11-H), 1.73 (1H, *m*, C10-H), 1.93 (1H, *m*, C10-H), 2.75 (6H, *s*, -CH₃), 2.93 (1 H, *m*, C7-H), 3.12 (3H, *m*, C2-H, C1'-H), 3.28 (1 H, *m*, C6-H), 3.37 (3H, *s*, -OCH₃), 3.63 (2H, *t*, *J*=6.5, C2'-H), 6.04 (2H, *m*, C8-H, C9-H), 10.21 (1H, *s*, HCl); MS (m/z): 100% = 279.2 [L+H⁺], 45% = 301.2 [L+Na⁺].

**Example XX (b2)**

**R₁= -OCH₃, R₂ = R₃ = R₃ₐ = R₄ = R₅ = R₆= -H, R= -CH₂N(C₂H₅)₂**

Yield: 75 %; white powder, m.p. 146.6-148.7 °C (from EtOH/(Et)₂O); ¹H NMR (300MHz, DMSO-d₆+ TMS, δ/ppm): 1.30 (6H, *t*, *J*=7.2, -CH₃), 1.40 (1H, *m*, C11-H), 1.51 (1H, *m*, C11-H), 1.80 (1H, *m*, C10-H), 1.97 (1H, *m*, C10-H), 3.04 (1 H, *m*, C7-H), 3.18 (8H, *m*, C2-H, C6-H, C1'-H, -CH₂-), 3.47 (3H, *s*, -OCH₃), 3.75 (2H, *t*, *J*=7.0, C2'-H), 6.10 (1H, *s*, C9-H), 6.11 (1H, *m*, C8-H),; MS (m/z): 100% = 307.2[L+H⁺], 62% = 329.2 [L+Na⁺].

**Example XXI (b3)**

**R₁= -OCH₃, R₂ = R₃ = R₃ₐ = R₄ = R₅ = R₆= -H, R= -CH₂NC₄H₈O**

Yield: 86 %; white powder, m.p. 190-192 °C (from EtOH/(Et)₂O); ¹H NMR (300MHz, DMSO-d₆+ TMS, δ/ppm): 1.40 (1H, *m*, C11-H), 1.53 (1H, *m*, C11-H), 1.80 (1 H, *m*, C10-H), 1.95 (1 H, *m*, C10-H), 3.04 (1H, *m*, C7-H), 3.16 (2H, *m*, C2-H, C6-H), 3.31 (4H, *m*, C1'-H, H-morph.), 3.37 (2H, *m*, H-morph.) 3.48 (3H, *s*, -OCH₃), 3.80 (2H, *t*, *J*=7.0, C2'-H), 3.94 (4H, *m*, H-morph.), 6.11 (2H, *m*, C8-H, C9-H); MS (m/z): 100% = 321.2[L+H⁺], 22% = 343.2 [L+Na⁺].

**Example XXII (b4)**

**R₁= -OCH₃, R₂ = R₃ = R₃ₐ = R₄ = - R₅ = R₆= -H, R= -CH₂NC₅H₁₀**

Yield: 96 %; white powder, m.p. 121.5-122.3 °C (from EtOH/(Et)₂O); ¹H NMR (300MHz, DMSO-d₆+ TMS, δ/ppm): 1.39 (1H, *m*, C11-H), 1.51 (1 H, *m*, C11-H), 1.67 (2H, *m*, H-piper.), 1.79 (4H, *m*, H-piper.), 1.94 (2H, *m*, C10-H), 3.04 (1 H, *m*, C7-H), 3.17 (4H, *m*, C2-H, C6-H, H-piper.), 3.34 (2H, *m*, C1'-H), 3.47 (3H, *s*, -OCH₃), 3.78 (2H, *t*, *J*=6.5, C2'-H), 6.09 (1 H, *s*, C9-H), 6.10 (1 H, *m*, C8-H); MS (m/z): 100% = 319.2[L+H⁺], 12% = 341.2 [L+Na⁺].

**Example XXIII (b5)**

**R₁= -OCH₃, R₂ = R₃ = R₃ₐ = R₄ = R₅ = R₆= -H, R= -(CH₂)₂N(CH₃)₂**

Yield: 89 %; white powder, m.p. 235-236 °C (from EtOH/(Et)₂O); ¹H NMR (300MHz, DMSO-d₆+ TMS, δ/ppm): 1.20 (1H*, m*, C11-H), 1.38 (1 H, *m*, C11-H), 1.74 (3H, *m*, C10-H, C2'-H), 1.91 (1H, *m*, C10-H), 2.70 (6H, *m*, -CH₃) 2.91 *(3H, m*, C7-H, C1'-H), 3.04 (1 H, *dd, J*=11.1, *J*=8.1, C6-H) 3.25 (1 H, *m*, C2-H), 3.31 (2H, *m*, C3'-H), 3.37 (3H, s, -OCH₃), 6.09 (2H, *m*, C9-H, C8-H), 10.08 (1 H, *m*, HCl); MS (m/z): 100% = 293.2[L+H⁺], 20% = 315.2 [L+Na⁺].

**Example XXIV (b6)**

**R₁= -OAc, R₂ = H, R₃ = -H, R₃ₐ = -H, R₄ = -OAc, R₅ = -H, R₆= -H, R= -CH ₂N(CH₃)₂**

Yield: 72 %; white powder, m.p. 249-251.4 °C (from EtOH/(Et)₂O); ¹H NMR (300MHz, DMSO-d₆+ TMS, δ/ppm): 1.71 (2H, *m*, C10-H, C11-H), 2.09 (6H, *s*, -OAc), 2.43 (2H, *m*, C10-H, C11-H), 2.74 (6H, *s*, -CH₃), 3.14 (2H, *m*, C1'-H), 3.65 (2H, *t*, *J*=6.1, C2'-H), 4.01 (2H, s, C2-H, C6-H), 6.14 (2H, *s*, C8-H, C9-H), 10.03 (1H, *s*, HCl); MS (m/z): 22% = 365.2[L+H⁺], 100% = 387.2 [L+Na⁺].

**Example XXV (b7)**

**R₁= -OAc, R₂ = H, R₃ = -H, R₃ₐ = -H, R₄ = -OAc, R₅ = -H, R₆= -H, R= -CH ₂N(C₂H₅)₂**

Yield: 79 %; white powder, m.p. 188.5-189 °C (from EtOH/(Et)₂O); ¹H NMR (300MHz, DMSO-d₆+ TMS, δ/ppm): 1.18 (6H, *t*, *J*=7.2, -CH₃), 1.72 (2H, *m*, C10-H, C11-H), 2.09 (6H, *s*, -OAc), 2.41 (2H, *m*, C10-H, C11-H), 3.11 (6H, *m*, C1'-H, -CH₂-), 3.67 (2H, *t*, *J*=6.6, C2'-H), 4.00 (2H, *s*, C2-H, C6-H), 6.15 (2H, *s*, C8-H, C9-H), 10.44 (1 H, *s*, HCl); MS (m/z): 100% = 393.2[L+H⁺], 79% = 415.2 [L+Na⁺].

**Example XXVI (b8)**

**R₁= -OAc, R₂ = H, R₃ = -H, R₃ₐ = -H, R₄ = -OAc, R₅ = -H, R₆= -H, R= -CH ₂NC₄H₈O**

Yield: 82 %; white powder, m.p. 232-236 °C (from EtOH/(Et)₂O); ¹H NMR (300MHz, DMSO-d₆+ TMS, δ/ppm): 1.72 (2H, *m*, C10-H, C11-H), 2.09 (6H, *s*, -OAc), 2.41 (2H, *m*, C10-H, C11-H), 3.03 (2H, *m*, H-morph.), 3.17 (2H, *m*, C1'-H), 3.39 (2H, *m*, H-morph.), 3.72(4H, *m*, C2'-H, H-morph.), 3.92 (2H, *m*, H-morph.), 4.01 (2H, *s*, C2-H, C6-H), 6.15 (2H, *s*, C8-H, C9-H), 11.22 (1 H, *s*, HCl); MS (m/z): 100% = 429.2 [L+Na⁺].

**Example XXVII** (b **9)**

**R₁= -OAc, R₂ = H, R₃ = -H, R₃ₐ = -H, R₄ = -OAc, R₅ = -H, R₆= -H, R= -CH ₂NC₅H₁₀**

Yield: 86 %; white powder, m.p. 269-272 °C (from EtOH/(Et)₂O); ¹H NMR (300MHz, DMSO-d₆+ TMS, δ/ppm): 1.34 (1H, *m*, H-piper.), 1.71 (7H, *m*, C10-H, C11-H, H-piper.), 2.09 (6H, *s,* -OAc), 2.41 (2H, *m,* C10-H, C11-H), 2.83 (2H, *m,* H-piper.), 3.09 (2H, *m,* C1'-H), 3.41 (2H, *m,* H-piper.), 3.69 (2H, *t, J*=5.5, C2'-H), 4.00 (2H, *s,* C2-H, C6-H), 6.14 (2H, *s,* C8-H, C9-H), 10.18 (1 H, *s,* HCl); MS (m/z): 100% = 405.2[L+H⁺], 69% = 427.2 [L+Na⁺].

**Example XXVIII (b10)**

**R₁= -OAc, R₂ = H, R₃ = -H, R₃ₐ = -H, R₄ = -OAc, R₅ = -H, R₆= -H, R= -(CH2)2N(CH3)2**

Yield: 82 %; white powder, m.p. 239-241 °C (from EtOH/(Et)₂O); ¹H NMR (300MHz, DMSO-d₆+ TMS, δ/ppm): 1.75 (4H, *m,* C10-H, C11-H, C2'-H), 2.09 (6H, *s,* -OAc), 2.40 (2H, *m,* C10-H, C11-H), 2.71 (6H, *s,* -CH₃), 2.91 (2H, *m,* C1'-H), 3.34 (2H, *m,* C3'-H), 3.95 (2H, *s,* C2-H, C6-H), 6.19 (2H, *s,* C8-H, C9-H), 9.84 (1 H, *s,* HCl); MS (m/z): 72% = 379.2[L+H⁺], 100% = 401.2 [L+Na⁺].

**Example XXIX (b11)**

**R₁= -CH₃, R₂ = H, R₃ = -CH₃, R₃ₐ= -CH₃, R₄ = -H, R₅ =-CH₃, R₆= -H, R= -CH₂N(CH₃)₂**

Yield: 76 %; white powder, m.p. 212-214 °C (from EtOH/(Et)₂O); ¹H NMR (300MHz, DMSO-d₆+ TMS, δ/ppm): 0.82 (3H, *s,* -CH₃), 0.89 (1 H, *m,* C10-H), 1.06 (3H, *s,* -CH₃), 1.21 (1 H, *m,* C10-H), 1.29 (3H, *s,* -CH₃), 1.65 (3H, *d J*=1.5, -CH₃), 2.33 (1 H, *m,* C7-H), 2.60 (1 H, *m,* C2-H), 2.76 (6H, *s,* -CH₃), 3.09 (2H, *m,* C1'-H), 3.24 (1 H, *dd*, *J*=11.1, *J*=7.8, C6-H), 3.62 (2H, *t* , J=6.3, C2'-H), 5.37 (1 H, *s,* C9-H), 9.74 (1 H, *s,* HCl); MS (m/z): 100% = 305.2 [L+H⁺], 31 % = 327.2 [L+Na⁺].

**Example XXX (b12)**

**R₁= -CH₃, R₂ = H, R₃ = R₃ₐ= -CH₃, R₄ = -H, R₅ = -CH₃, R₆= -H, R= -CH₂ N(C₂H₅)₂**

Yield: 89 %; white powder, m.p. 128-130 °C (from EtOH/(Et)₂O); ¹H NMR (300MHz, DMSO-d₆+ TMS, δ/ppm): 0.82 (3H, *s,* -CH₃), 0.90 (1 H, *m,* C10-H), 1.05 (3H, *s,* -CH₃), 1.19 (7H, *m,* C10-H, -CH₃), 1.28 (3H, *s,* -CH₃), 1.65 (3H, *d*, *J*=1.5, -CH₃), 2.32 (1 H, *m,* C7-H), 2.61 (1 H, *m,* C2-H), 3.02 (2H, *m,* C1'-H), 3.12 (4H, *m,* -CH₂-), 3.29 (1H, *dd*, *J*=11.1, *J*=7.8, C6-H), 3.65 (2H, *t*, *J*=6.9, C2'-H), 5.38 (1 H, s, C9-H), 10.59 (1 H, *s,* HCl); MS (m/z): 100% = 333.2 [L+H⁺].

**Example XXXI (b13)**

**R₁= -CH₃, R₂ = H, R₃ = R₃ₐ= -CH₃, R₄ = -H, R₅ = -CH₃, R₆= -H, R= -CH₂ NC₄H₈₀**

Yield: 86 %; white powder, m.p. 203-206 °C (from EtOH/(Et)₂O); ¹H NMR (300MHz, DMSO-d₆+ TMS, δ/ppm): 0.82 (3H, *s,* -CH₃), 0.90 (1 H, *m,* C10-H), 1.06 (3H, *s*, -CH₃), 1.21 (1 H, *m,* C10-H), 1.29 (3H, *s,* -CH₃), 1.66 (3H, *d J*=1.5, -CH₃), 2.32 (1H, *m,* C7-H), 2.63 (1H, *m,* C2-H), 3.13 (4H, *m,* H-morph.), 3.26 (1 H, *m,* C6-H), 3.41 (2H, *m,* C1'-H), 3.67 (4H, *m,* H-morph.), 3.94 (2H, *m,* C2'-H), 5.38 (1H, *s,* C9-H), 10.60 (1H, *s,* HCl); MS (m/z): 100% = 347.2 [L+H⁺], 89% = 369.2 [L+Na⁺].

**Example XXXII (b14)**

**R₁= -CH₃, R₂ = H, R₃ = R₃ₐ= -CH₃, R₄ = -H, R₅ = -CH₃, R₆= -H, R= -CH₂ NC₅H₁₀**

Yield: 89 %; white powder, m.p. 143-145 °C (from EtOH/(Et)₂O); ¹H NMR (300MHz, DMSO-d₆+ TMS, δ/ppm): 0.82 (3H, *s,* -CH₃), 0.90 (1 H, *m,* C10-H), 1.06 (3H, *s,* -CH₃), 1.21 (1 H, *m,* C10-H), 1.29 (3H, *s,* -CH₃), 1.60 (1 H, *m,* H-piper.), 1.65 (5H, *m*, -CH₃, H-piper.), 1.77 (2H, *m,* H-piper.), 2.32 (1 H, *m,* C7-H), 2.61 (1 H, *m,* C2-H), 2.87 (2H, *m,* H-piper.), 3.04 (2H, *m,* C1'-H), 3.25 (2H, *m,* C6-H, H-piper.), 3.44 (2H, *m,* H-piper.), 3.66 (2H, *t* , *J*=6.9, C2'-H), 5.38 (1 H, *s*, C9-H), 9.42 (1 H, *s,* HCl); MS (m/z): 100% = 345.2 [L+H⁺].

**Example XXXIII (b15)**

**R₁= -CH₃, R₂ = H, R₃ = R₃ₐ= -CH₃, R₄ = -H, R₅ = -CH₃, R₆= -H, R= -(CH₂ )₂N(CH₃)₂**

Yield: 78 %; white powder, m.p. 185-186.3 °C (from EtOH/(Et)₂O); ¹H NMR (300MHz, DMSO-d₆+ TMS, δ/ppm): 0.82 (3H, *s,* -CH₃), 0.90 (1 H, *m,* C10-H), 1.06 (3H, *s,* -CH₃), 1.19 (1 H, *m,* C10-H), 1.28 (3H, *s,* -CH₃), 1.68 (3H, *d, J=*1.5, -CH₃), 1.75 (2H, *m,* C2'-H), 2.33 (1 H, *m,* C7-H), 2.55 (1 H, *m* , C2-H), 2.71 (6H, *s,* -CH₃), 2.89 (2H, *m,* C1'-H), 3.23 (1H, *dd*, *J=*11.1, *J* =7.8, C6-H), 3.34 (2H, *m,* C3'-H), 5.42 (1 H, *s,* C9-H), 9.89 (1 H, *s*, HCl);
MS (m/z): 100% = 319.2 [L+H⁺].

**Example XXXIV (c1)**

**R₁= -OCH₂CH(CH₃)₂, R₂ = -H, R= -CH₂N(CH₃)₂**

Yield: 89 %; white powder, m.p. 90-93 °C (from EtOH/(Et)₂O); ¹H NMR (300MHz, DMSO-d₆+ TMS, δ/ppm): 0.89 (6H, *dd, J*=9.3, *J*=6.6, -CH₃), 1.60 (1 H, *m,* C10-H), 1.76 (2H, *m,* C10-H, C11-H), 2.04 (2H, *m,* C11-H, -CH-), 2.44 (1 H, *m,* C9-H), 2.56 (1 H, *m,* C9-H), 2.77 (6H, *m*, -CH₃), 3.21 (3H, *m,* C6-H, C1'-H), 3.32 (2H, *m,* C2-H, C7-H), 3.43 (2H, *m,* -CH₂-), 3.69 (2H, *m,* C2'-H), 10.07 (1 H, *s,* HCl); MS (m/z): 100% = 337.3 [L+H⁺].

**Example XXXV (c2)**

**R₁= -OCH₂CH(CH₃)₂, R₂ = -H, R= -CH₂N(C₂H₅)₂**

Yield: 91 %; white powder, m.p. 155-158 °C (from EtOH/(Et)₂O); ¹H NMR (300MHz, DMSO-d₆+ TMS, δ/ppm): 0.89 (6H, *dd, J*=9.3, *J*=6.6, -CH₃), 1.19 (6H, *m,* -CH₃), 1.60 (1 H, *m,* C10-H), 1.75 (2H, *m,* C10-H, C11-H), 2.03 (2H, *m,* C11-H, -CH-), 2.44 (1H, *m,* C9-H), 2.56 (1H, *m,* C9-H), 3.15 (7H, *m,* C6-H, C1'-H, -CH₂-), 3.31 (1H, *m,* C7-H), 3.41 (3H, *m,* -C2-H, -CH ₂-), 3.69 (2H, *m,* C2'-H), 10.08 (1 H, s, HCl); MS (m/z): 100% = 365.3 [L+H ⁺], 19% = 387.3 [L+Na⁺].

**Example XXXVI (c3)**

**R₁= -OCH₂CH(CH₃)₂, R₂ = -H, R= -CH₂NC₄H₈₀**

Yield: 89 %; white powder, m.p. 238-241.7 °C (from EtOH/(Et)₂O); ¹H NMR (300MHz, DMSO-d₆+ TMS, δ/ppm): 0.94 (6H, *m,* -CH₃), 1.79 (1 H, *m* , C10-H), 1.96 (2H, *m,* C10-H, C11-H), 2.11 (1H, *m,* C11-H) 2.20 (1 H, *m,* -CH-), 2.54 (1 H, *m,* C9-H), 2.71 (1 H, *m,* C9-H), 3.07 (1 H, *m,* H-morph.), 3.21 (4H, *m,* H-morph.), 3.40 (3H, *m,* H-morph.), 3.53 (3H, *m,* C6-H, C1'-H), 3.83 (4H, *m,* C2-H, C7-H, -CH₂-), 4.06 (2H, *m,* C2'-H); MS (m/z): 28% = 379.3 [L+H⁺], 100% = 401.3 [L+Na⁺].

**Example XXXVII (c4)**

**R₁= -OCH₂CH(CH₃)₂, R₂ = -H, R= -CH₂NC₅H₁₀**

Yield: 88 %; white powder, m.p. 212-214 °C (from EtOH/(Et)₂O); ¹H NMR (300MHz, DMSO-d₆+ TMS, δ/ppm): 0.94 (6H, *m,* -CH₃), 1.79 (3H, *m,* C10-H, H-piper.), 1.96 (4H, *m,* C10-H, C11-H, H-piper.), 2.06 (1H, *m,* -CH-), 2.15 (2H, *m,* H-piper.), 2.55 (1 H, *m,* C9-H), 2.70 (1 H, *m,* C9-H), 3.07 (1 H, *m,* H-piper.), 3.24 (3H, *m,* H-piper.), 3.41 (3H, *m,* C6-H, C1'-H), 3.52 (4H, *m,* C2-H, C7-H, -CH₂-), 3.84 (2H, *m,* C2'-H); MS (m/z): 100% = 377.4 [L+H⁺].

**Example XXXVIII (c5)**

**R₁= -OCH₂CH(CH₃)₂, R₂ = -H, R= -(CH₂)₂N(CH₃)₂**

Yield: 84 %; white powder, m.p. 52-55 °C (from EtOH/(Et)₂O); ¹H NMR (300MHz, DMSO-d₆+ TMS, δ/ppm): 0.89 (6H, *dd, J*=9.6, *J*=6.6, -CH₃), 1.73 (4H, *m,* C10-H, C10-H, C11-H, C2'-H), 2.04 (2H, *m,* C11-H, -CH-), 2.55 (2H, *m,* C9-H), 2.72 (6H, *s,* -CH₃), 2.86 (2H, *m,* C1'-H), 3.21 (1 H, *m,* C6-H), 3.36 (6H, *m,* C2-H, C7-H, -CH₂-, C3'-H), 9.74 (1 H, *s,* HCl); MS (m/z): 100% = 351.2 [L+H⁺].

**Example XXXIX (c6)**

**R₁= -CH₃, R₂ = -OCH₂CH(CH₃)₂, R= -CH₂N(CH₃)₂**

Yield: 87 %; white powder, m.p. 231.6-233.7 °C (from EtOH/(Et)₂O); ¹H NMR (300MHz, DMSO-d₆+ TMS, δ/ppm): 1.20 (3H, *t, J*= 7.0, -CH₃), 1.25 (3H, *s,* -CH₃), 1.86 (2H, *m,* C11-H), 2.30 (1 H, *m,* C9-H), 2.54 (1 H, *m,* C10-H), 2.66 (1 H, *m,* C9-H), 2.76 (7H, *m,* C7-H, -CH₃), 3.00 (1 H, *dd*, *J* =11.4, *J*=9.3, C2-H), 3.18 (2H, *m,* C1'-H), 3.39 (1H, *dd, J*=12.9, *J*=9.6, C6-H), 3.69 (2H, *m,* C2'-H), 4.11 (2H, *m*, -CH₂-), 10.26 (1H, *s*, HCl); MS (m/z): 32% = 351.2 [L+H⁺], 100% = 373.2 [L+Na⁺].

**Example XL (c7)**

**R₁= -CH₃, R₂ = -OCH₂CH(CH₃)₂, R= -CH₂N(C₂H₅)₂**

Yield: 88 %; white powder, m.p. 185-187.6 °C (from EtOH/(Et)₂O); ¹H NMR (300MHz, DMSO-d₆+ TMS, δ/ppm): 1.20 (9H, *m,* -CH₃), 1.25 (3H, *s,* -CH₃), 1.86 (2H, *m,* C11-H), 2.28 (1 H, *m,* C9-H), 2.54 (1 H, *m,* C10-H), 2.66 (1 H, *m,* C7-H), 2.77 (1 H, *m,* C9-H), 3.00 (1 H, *m,* C2-H), 3.13 (6H, *m,* C1'-H, -CH₂-), 3.39 (1H, *m,* C6-H), 3.70 (2H, *m,* C2'-H), 4.11 (2H, *m*, -CH₂ -), 10.36 (1 H, *s,* HCl); MS (m/z): 100% = 379.2 [L+H⁺], 22% = 568.4 [L+Na ⁺].

**Example XLI (c8)**

**R₁= -CH₃, R₂ = -OCH₂CH(CH₃)₂, R= -CH₂NC₄H₈₀**

Yield: 76 %; white powder, m.p. 235.4-238.7 °C (from EtOH/(Et)₂O); ¹H NMR (300MHz, DMSO-d₆+ TMS, δ/ppm): 1.20 (3H, *t*, *J*= 7.0, -CH₃), 1.25 (3H, *s,* -CH₃), 1.86 (2H, *m,* C11-H), 2.29 (1 H, *m,* C9-H), 2.66 (1 H, *m,* C10-H), 2.76 (1 H, *m,* C9-H), 3.02 (2H, *m,* C2-H, C7-H), 3.24 (2H, *m,* H-morph.), 3.42 (3H, *m,* C6-H, C1'-H), 3.73 (4H, *m,* H-morph.), 3.92 (2H, *m,* C2'-H), 4.11 (2H, *m,* -CH₂-), 11.26 (1 H, *s,* HCl); MS (m/z): 5% = 392.2 [L+H⁺], 100% = 415.2 [L+Na⁺].

**Example XLII (c9)**

**R₁= -CH₃, R₂ = -OCH₂CH(CH₃)₂, R= -CH₂NC₅H₁₀**

Yield: 83 %; white powder, m.p. 210-213 °C (from EtOH/(Et)₂O); ¹H NMR (300MHz, DMSO-d₆+ TMS, δ/ppm): 1.20 (3H, *t*, *J*= 7.0, -CH₃), 1.24 (3H, *s,* -CH₃), 1.34 (1H, *m,* H-piper.), 1.76 (7H, *m,* C11-H, H-piper.), 2.28 (1H, *m,* C9-H), 2.66 (1 H, *m,* C10-H), 2.81 (3H, *m,* C9-H, H-piper.), 3.00 (1 H, *dd*, *J* =11.1, *J*=9.0, C2-H), 3.13 (2H, *m,* C1'-H), 3.43 (3H, *m,* C6-H, H-piper.), 3.71 (2H, *m,* C2'-H), 4.13 (2H, *m*, -CH₂-), 10.06 (1H, *s,* HCl); MS (m/z): 100% = 391.3 [L+H⁺].

**Example XLIII (c10)**

**R₁= -CH₃, R₂ = -OCH₂CH(CH₃)₂, R= -(CH₂)₂N(CH₃)₂**

Yield: 86 %; white powder, m.p. 47-50 °C (from EtOH/(Et)₂O); ¹H NMR (300MHz, DMSO-d₆+ TMS, δ/ppm): 1.21 (3H, *t, J*= 7.2, -CH₃), 1.26 (3H, *s,* -CH₃), 1.83 (4H, *m,* C11-H, C2'-H), 2.29 (1 H, *m,* C9-H), 2.63 (2H, *m,* C9-H, C10-H), 2.72 (6H, *s,* -CH₃), 2.85 (4H, *m,* C2-H, C7-H, C1'-H), 3.32 (1 H, *m,* C6-H), 3.38 (2H, *m,* C3'-H), 4.11 (2H, *m,* -CH₂-); MS (m/z): 100% = 365.2 [L+H⁺].

Industrial applicability

Example XLIV

**Cytotoxic properties of derivatives of dicarboximides**

A series of dicarboximides derivatives (table 1) was tested for cytotoxic properties against four cell lines. The tests were conducted by means of MTT assay where yellow tetrazole salt was processed by the dehydrogenases in mitochondria of live cells into violet formazan. The following cell lines were used:

- HeLa cells (uterine cervix cancer)

- HL60 cells (human acute myelogenous leukaemia)

- K562 cells (human chronic myelogenous leukaemia) and

- HUVEC cells (normal human endothelial cells).

1. HeLa, K562, HL60, HUVEC cells in an amount of 7 thousand cells / well (in 200 µL medium) were seeded in 96-well plates. Cells were incubated overnight in an incubator (37°C, 5% CO2)

2. Test compounds were dissolved in DMSO and added in an amount of 2µL to the surface of the cells to give the following final concentrations in the cell culture (1 mM, 10 µm, 100 nM, 1 nM). As a control, cells exposed to 1% DMSO were used.

3. Cytotoxicity of the test compounds was evaluated by MTT test. After 48-hour incubation of HeLa, K562, HL60 or HUVEC cells with the test compounds, MTT (5mg/mL) was added in an amount of 25µL/well. MTT, a yellow tetrazolium salt was reduced to purple formazan by mitochondrial dehydrogenases of living cells. The amount of formazan could be determined spectrophotometrically and was proportional to the number of live cells. Cells were incubated with MTT for 2 hours in an incubator (37°C, 5% CO₂). Next, lysis buffer containing SDS and DMF was added (95µL/well) to each well and incubated overnight at 37°C, 5% CO₂.

4. The plates were analyzed by reading the absorbance at a wavelength of 570 nm and 650 nm using the plate-reader Fluostar Omega. The measured absorbance at 570 nm is proportional to the number of living cells. IC₅₀ values (concentration of compound causing 50% cell mortality) were read from the charts by interpolation to 50% cell survival.

The results (shown in the table 1) indicate a high and selective toxicity of compounds **a3-7** against the K562 and HL60 cells of chronic and acute myelogenous leukaemia. Calculated IC₅₀ values for these compounds are within the range from 1 to 10 µM.

**TABLE 1**

**Table 1**

| **compound** | **HeLa** | **K562** | **HUVEC** | **HL-60** |
|---|---|---|---|---|
| | **IC50 48h** | **IC50 48h** | **IC50 48h** | **IC50 48h** |
| **a1** | 1 µM* | 1 µM* | > 1 mM* | |
| **a2** | 1 µM* | 1 µM* | 4 µM* | |
| **a3** | > 1 mM* | 10 µM* | > 1 mM* | > 1 mM* |
| | > 1mM* | | > 1 mM* | > 1 mM* |
| **a4** | > 1 mM* | 4.5 µM* | > 1 mM* | > 1 mM* |
| **a5** | > 1 mM* | 2 µM* | > 1 mM* | > 1 mM* |
| | > 1mM* | | | |
| **a6** | > 1 mM* | 8 µM* | > 1 mM* | > 1 mM* |
| **a7** | > 1 mM* | 1 µM* | > 1 mM* | 2 µM |
| **a8** | > 1 mM* | > 1 mM* | | |
| **a9** | 1 µM* | 1 µM* | 1 µM* | |
| **a10** | 1 µM* | 1 µM* | 1 µM* | |
| **a11** | 90 µM | 90 µM | 70 µM | |
| **a12** | 100 µM | 90 µM | 100 µM | |
| **a13** | 1 µM* | 0,9 µM* | 2 µM* | |
| **a14** | 1 µM* | 0,9 µM* | 2 µM* | |
| **b1** | > 1 mM | > 1 mM | | |
| **b2** | > 1 mM | > 1 mM | | |
| **b3** | > 1 mM | > 1 mM | | |
| **b4** | > 1 mM | > 1 mM | | |
| **b5** | > 1 mM | > 1 mM | | |
| **b6** | > 1 mM | > 1 mM | | |
| **b7** | > 1 mM | > 1 mM | | |
| **b8** | > 1 mM | > 1 mM | | |
| **b9** | > 1 mM | > 1 mM | | |
| **b10** | > 1 mM | > 1 mM | | |
| **b11** | 90 µM | 100 µM | 300 µM | |
| **b12** | 1000 µM | > 1 mM | | |
| **b13** | 90 µM | 90 µM | 100 µM | |
| **b14** | 100 µM | 100 µM | | |
| **b15** | 100 µM | 90 µM | 700 µM | |
| **c1** | > 1 mM | > 1 mM | | |
| **c2** | > 1 mM | > 1 mM | | |
| **c3** | > 1 mM | > 1 mM | | |
| **c4** | > 1 mM | > 1 mM | | |
| **c5** | > 1 mM | > 1 mM | | |
| **c6** | 100 µM | > 1 mM | > 1 mM | |
| **c7** | > 1 mM | > 1 mM | | |
| **c8** | > 1 mM | > 1 mM | | |
| **c9** | > 1 mM | > 1 mM | | |
| **c10** | > 1 mM | > 1 mM | | |

COMMENTS:

* compounds precipitated at a concentration of 1 mM from cell cultures

Test compounds were neither toxic to uterine cervix cancer cells (HeLa) nor normal HUVEC cells (umbilical vein endothelium). The above mentioned results imply that compounds **a3-a7** are **selectively** toxic to the neoplasms of leukaemia type and **are not toxic to normal cells.**

Compounds **a3-6** are toxic only to chronic myelogenous leukaemia cells (K562), however they are not toxic to acute promyelocytic leukaemia cells (HL60), uterine cervix cancer cells (HeLa) and normal cells (HUVEC).

Compound **a7** is specifically toxic to leukaemia cells K562 and HL60, hovewer it is not toxic to uterine cervix cancer cells (HeLa) and normal cells (HUVEC).

**Example L. - Mechanism of Toxicity of dicarboximide derivatives.**

The observed cytotoxicity of compounds **a3-7** was tested for its origin, i.e. induction of necrosis or apoptosis in the K562 cells of chronic myelogenous leukaemia.

K562 cells in RPMI 1640 medium containing 10% fetal calf serum and antibiotics were seeded on 96-well plate in an amount of 20x10³ cells / well. Cells were cultured for 24 hours in an incubator (5% CO₂, 37°C).

Compounds a3-a7 were dissolved in DMSO and added to the cell medium; final concentration in the cell culture was 5 x IC₅₀. Cells cultured in the presence of 1% DMSO served as a negative control. Cells grown in the presence of 1 µM staurosporin (a strong inducer of apoptosis) served as a positive control. The cells were incubated with the compounds for 18 hours in the incubator (5% CO₂; 37°C).

The activity of caspase 3 and 7 was determined by means of Apo-ONE^{®} Homogeneous Caspase-3/7 Assay (Promega, Madison, WI, USA), in compliance with manufacturer's guidelines. Cells were lysed and incubated with a profluorescent substrate for caspases for 1.5 hours at room temperature. Due to proteolytic activity of caspases, the profluorescent substrate was converted into a fluorescent product the amount of which could be determined by measurement of fluorescence intensity.

The fluorescence intensity in each well was measured using a plate reader Fluostar Omega (BMG-Labtech, Germany) at an excitation wavelength of 485 nm and emission 520 nm. The intensity of fluorescence is proportional to the activity of caspase 3 and 7 in the sample.

**Results**

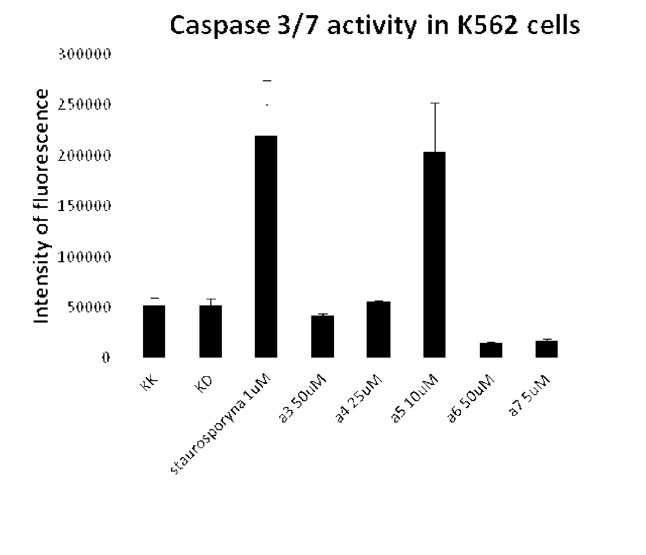

We have observed (Fig. 1) that compared with reference cells (K562 and K562+DMSO), the activity of caspase 3 and 7 was increased in chronic myelogenous leukaemia (K562) cells incubated with compound **a5** at the concentration of 10 µM. This indicates that the cytotoxicity of compound **a5** results from the induction of apoptosis in the chronic myelogenous leukaemia (K562) cells. The compounds **a3, a4, a6, a7** do not increase the activity of caspases 3 and 7, so their cytotoxicity results from the induction of necrosis rather than apoptosis in the K562 cells.

### Citation list

1. Saez R, Craig J, Kuhn J, et al: Phase I clinical investigation of amonafide. J Clin Oncol 7: 1351-1358, 1989

2. Llombart M, Poveda A, Forner E, et al: Phase I study of mitonafide in solid tumors. Invest New Drugs 10: 177-181, 1992

3. Wu A, Liu J, Qin S, Mei P: Monatsh Chem 141: 95-99, 2010

4. Mukherjee A, et al: Journal of Experimental and Clinical Cancer Research 29: 175, 2010

5. Van Quaquebeke E, et al: J. Med. Chem 50 (17): 4122-4134, 2007

6. Chen Z, et al: J. Med. Chem 53 (6): 2589-2600, 2010

7. Xu G, et al: Letters in Drug Design & Discovery 6: 51-55, 2009

8. Faul M, et al: Bioorganic and Medicinal Chemistry Letters 13(11): 1857-1859, 2003

9. Sanchez-Martinez C, et al: Bioorganic and Medicinal Cemistry Letters 13: 3841-3846, 2003

10. Sodeoka M, et al: The Chemical Record 10: 308-314, 2010

11. Lan Li, et al: Molecular Pharmacology, 52: 798-806, 1997

12. Bachowska B, et al.: Chemistry Open 1: 33-38, 2012

## Claims

1. New, substituted derivatives of dicarboximides represented by the general formula 1: possibly in the form of pharmaceutically acceptable salts, wherein R mean the alkyl group C1-C6, optionally, the alkyl group is substituted -OH group or amine group, selected from group containing -NH-R₁ group or the group with formula **2** or **3**, where R₁,R₂,R₃ are the same or different and cover the alkyl group C1-C3 and R₄ means -CH₂- or -O- 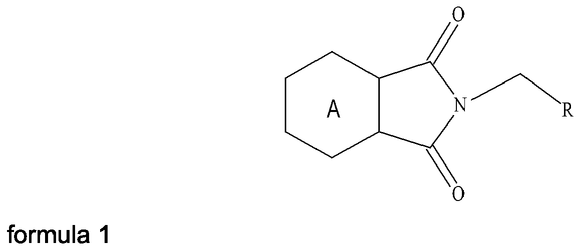 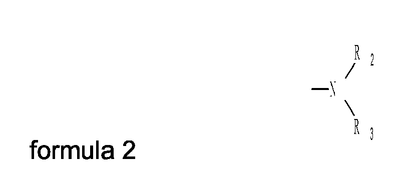 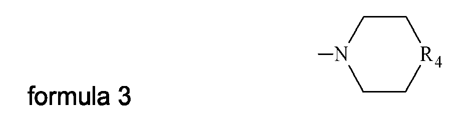 and combinations of these substituents, where **A** in Formula **1** means the structure represented by the general formula **a** 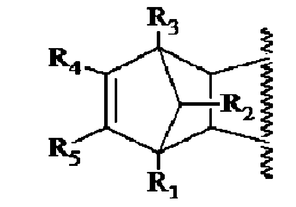 **formula a** wherein R₁, R₃, R₄, R₅ are the same or different and mean- the alkyl group-(CH₂)ₙCH₃ where n = 0 ÷ 6, or -C₆H₅ group and its derivatives selected from the group consisting halogen derivatives, hydroxyl derivatives, -O-alkyl type -O-(CH₂)ₙ-CH₃ where n = 0 ÷ 6, R₂ means a -H, =O, -OH, -(CH₂)ₙCH₃ type substituents where n = 0 ÷ 6 or -C₆H₅ group and its derivatives selected from the group consisting halogen derivatives, hydroxyl derivatives, -O-alkyl type -O-(CH₂)ₙ-CH₃ where n = 0 ÷ 6, or represented by the general formula b 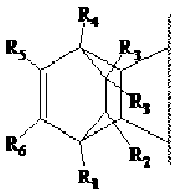 formula b wherein: R₁ means a -(CH₂)ₙCH₃, -O-(CH₂)ₙCH₃, -CO(CH₂)ₙCH₃ type substituents where, n = 0 ÷ 6, R₂, R₄ are the same or different and mean a -H, -(CH₂)ₙCH₃, -O-(CH₂)ₙCH₃ type substituents where n = 0 ÷ 6, R₃, R₃ₐ, R₅, R₆ are the same or different and mean a -H, -OH,-halogen, -(CH₂)ₙCH₃ type substituents where n = 0 ÷ 6, or represented by the general formula c 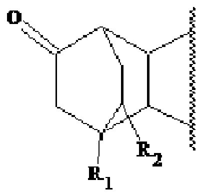 formula c wherein: R₁ means a -(CH₂)ₙCH₃, -O-(CH₂)ₙCH₃, -CO(CH₂)ₙCH₃ type substituents where n = 0 ÷ 6, R₂ means a -halogen, -(CH₂)ₙCH₃, -COO(CH₂)ₙCH₃ type substituents where n = 0 ÷ 6, and their salts showing biological activity.

2. Compounds according to claim. 1, **characterized in that** they are presented by the structures **a3, a4, a5, a6, a7**

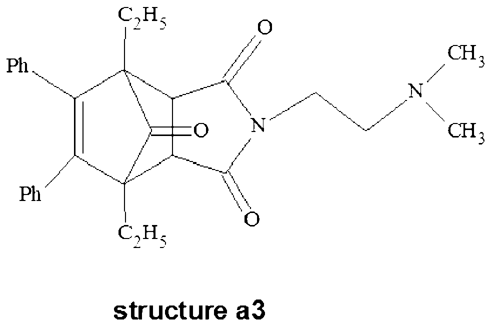

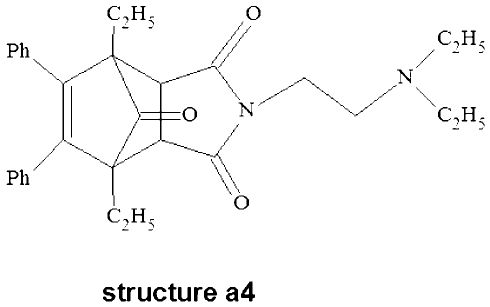

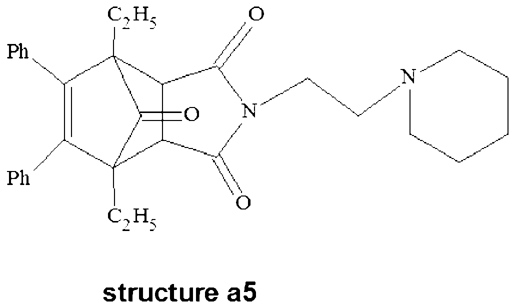

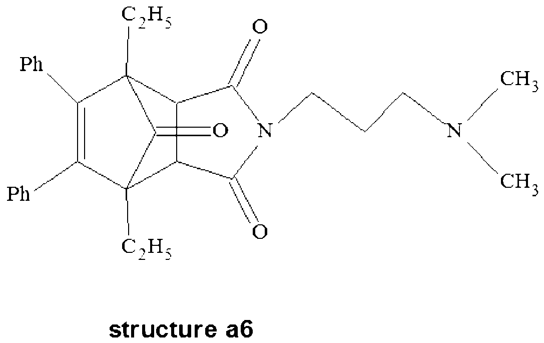

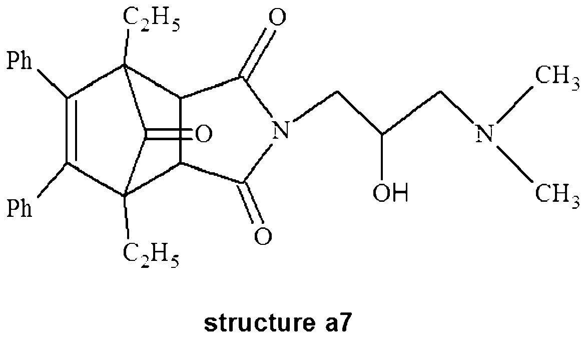

3. Use of compounds according to claim 1 and claim 2, optionally in the form of pharmaceutically approved salts for the manufacture of antiproliferative drugs for use in therapy of normal and neoplastic cells, either alone or in combination with already existing drugs.
